# NEUE EUROPÄISCHE PATENTSCHRIFT

(11) **EP 0 907 351 B2**
(45) Veröffentlichungstag und Bekanntmachung des Hinweises auf die Entscheidung über den Einspruch: **18.04.2012**
(45) Hinweis auf die Patenterteilung: 09.05.2001
(21) Anmeldenummer: 97903339.6
(22) Anmeldetag: 20.02.1997
(51) Int. Cl.: A61Q 19/06

(54) **KOSMETIKUM BZW. KOSMETIKZUSAMMENSETZUNG ZUR GLÄTTUNG UND STRAFFUNG DER HAUT BEI GESTÖRTEM UNTERHAUT-BINDE-FETTGEWEBE, INSBESONDERE BEI DER "CELLULITE"**
COSMETIC OR COSMETIC PREPARATION FOR SMOOTHING AND TIGHTENING THE SKIN IN THE CASE OF SUBCUTANEOUS FATTY TISSUE PROBLEMS, PARTICULARLY CELLULITE
COSMETIQUE OU PREPARATION COSMETIQUE POUR LE LISSAGE ET LA TENSION DE LA PEAU EN CAS D'AFFECTIONS DU TISSU ADIPEUX SOUS-CUTANE, EN PARTICULIER EN CAS DE CELLULITE

(30) Priorität: 29.03.1996 DE 19612748
(43) Veröffentlichungstag der Anmeldung: 14.04.1999
(73) Patentinhaber: Keyvest GmbH, 79232 March-Buchheim (DE)
(72) Erfinder: SCHMIDT, Alfred, D-22301 Hamburg (DE); WIELAND, Heinrich, D-79271 St. Peter (DE)
(74) Vertreter: Feldmeier, Jürgen
(86) Internationale Anmeldenummer: PCT/EP1997/000811
(87) Internationale Veröffentlichungsnummer: WO 1997/036570

(56) Entgegenhaltungen:
- EP-A- 0 240 131
- EP-A- 0 728 471
- EP-A1- 0 151 326
- WO-A1-96/08231
- DE-A1- 4 432 947
- FR-A- 2 735 687
- FR-A1- 2 571 616
- JOURNAL OF APPLIED COSMETOLOGY, Bd. 13, Nr. 4, Oktober 1995 - Dezember 1995, ROME, Seiten 220-220, XP000676633 M.ICARE,P.MORGANTI,L.TIBERI: "Anti-Cellulitis Transdermal Delivery System: Analysis of the Active Ingredients"
- SCHARZEL W.C. ET AL. ENDOCRINOLOGY Bd. 92, 1973, Seiten 866 - 880
- 'Römpp Chemie Lexikon, 9. Auflage', 1992
- MESSINA M. J. ET AL. NUTRITION AND CANCER Bd. 21, Nr. 2, 1994, Seiten 113 - 131
- 'Pschyrembel: Klinisches Wörterbuch', 1998

## Beschreibung

Die vorliegende Erfindung betrifft ein Verfahren zur kosmetischen Behandlung von Cellulite, bei dem eine Kosmetikzusammensetzung auf die zu behandelnde Haut appliziert wird.

Mittel und Wege zur Straffung und Glättung der Haut sind eine bedeutende kosmetische Herausforderung. Eine unerwünschte Folge der Fettgewebsbildung in der Haut ist insbesondere die Cellulite.
Cellulite ist eine Bezeichnung für eine nicht-entzündliche konstitutionell (geschlechtstypisch) bedingte Adipositas mit leichter Lymphstauung und geringer (mukoider) Ödembildung im Bereich des Bindegewebes (sogenannte Adipositas circumscripta oedematosa). Cellulite kommt besonders bei Frauen in der Hüft-, Oberschenkel- und Glutealregion vor. Meist ist ein sogenanntes "Matratzenphänomen" (durch Bindegewebssepten netzartig eingezogene Oberfläche) sowie das sogenannte "Orangenschalenhaut-Phänomen" (trichterförmige Follikeleinziehungen nach Kneifen) erkennbar. Dabei kommt es zu einer Bindegewebsstörung der Subcutis und zu einer Massezunahme der Lipide in den Fettkammern. Das Bild der Cellulite hat jedoch keinen Krankheitswert.

Einen Überblick über Definition, Erscheinungsbild und Therapieansätze gibt der Aufsatz von M. Rimpler in: "Biologische Medizin", 23. Jahrgang, Heft 5, Seiten 284-286 (1994). Dabei wird für das Auftreten der Cellulite eine Funktionsminderung des Gefäßsystems als wesentliche Schädigung verantwortlich gemacht. Grundlage für die vorgeschlagenen Behandlungsansätze bildet die Erkenntnis, daß die Mikrozirkulationsstörungen im Stadium der Cellulite in der Hautschicht alle Stoffwechselprozesse und Syntheseleistungen nachfolgender Zellpopulationen, besonders die der Fibroplasten im Corium und der epidermalen Zellen, beeinträchtigen. Ziel der Behandlungen muß es danach sein, eine ausreichende Vaskularisierung und Versorgung der Subcutis wiederherzustellen. Um dies zu erreichen, wurde ein Massagesystem entwickelt, bei dem eine physikalische Behandlung der Hautoberfläche mit Hilfe eines kleinen Massagegerätes unter gleichzeitigem Einsatz von ausgesuchten Phytoextrakten erfolgt (siehe M. Rimpler, "Die Dermapunkturfibel", 1. Auflage, S. 93-126, G.A. Ulmer Verlag, Tuningen (1993); Cellulite-Studie, Studienplan Nr. 1990-2, MHH OE 4330, Medizinische Hochschule Hannover (1990); und M. Rimpler, Chr. Rimpler und S. Lemke in: "Haut", Heft 3 (1994), Seiten 1-4.

Ein alternativer Ansatz der Kombination einer mechanischen und wirkstofflichen Einwirkung auf die von Cellulite betroffenen Hautpartien ist in dem US-Patent Nr. 4 829 987 offenbart. Dabei werden die jeweiligen Körperteile, insbesondere Oberschenkel, Hüfte und Gesäß, unter Anwendung spezieller Übungsgeräte einer dynamischen isometrischen Einwirkung unterworfen, während diese Körperteile einen Verband tragen, der mit einer Lösung eines mineralischen Stoffes, zum Beispiel Meerschlamm, getränkt ist. Mit dieser Behandlung sollen bestimmte Mineralien aus Fettablagerungen des Körpers extrahiert werden, um das Elastin des Weichgewebes zu revitalisieren.

Der Nachteil von solchen mechanischen Behandlungsmethoden ergibt sich daraus, daß ein durch kräftige Massagen erzeugter, externer Druck die Zellen irritiert und vielfältige Reaktionen der Hautzellen hervorruft. Als Folge produzieren die Zellen verstärkt Elastase und Collagenase. Diese Stützgewebe-abbauenden Enzyme lassen das Bindegewebe eher erschlaffen, als es zu straffen.

Neben Massagesystemen sind in jüngster Zeit Kosmetika auf den Markt gekommen, die angebliche Anti-Cellulite-Wirkstoffe enthalten, wie beispielsweise Seetang-Extrakt, Koffein, Theophylin oder fettabbauende Enzyme. Eine weitere Creme gegen Celulite enthält als Wirkstoffe Extrakte von Elizabethae, einer Korallenart und von Heidekraut, die Entzündungen im Gewebe und damit die Entstehung gewebeschwächender Enzyme bekämpfen sollen, sowie einen Algenbestandteil, der die Fettverbrennung aktivieren soll; daneben sollen Centella Asiatica, Milchproteine und Vitamin A die geschwächte Collagen- und Elastinproduktion stärken, und Fruchtsäuren sollen die Haut glätten.

Im "Journal of Applied Cosmetology", Bd. 13 (1995) S. 220 wird der experimentelle Versuch beschrieben, ein neues Applikationssystem, nämlich das sogenannte "transdermal delivery cosmetic system (TDCS)", zur Behandlung der Cellulite einzusetzen. Dabei wird der Wirkstoff mit einem Pflaster mit der betroffenen Hautpartie in Kontakt gebracht. Zur Untersuchung werden herkömmliche Cellulitemittel eingesetzt, unter anderem Fucosterol, welches aus der Meeresalge (Tang) "Quercia Marina" isoliert wurde. Daneben werden noch Triterpen-Saponine sowie aus der "Centella asiatica L."-Pflanze extrahiertes Aglycon eingesetzt.

Die FR-A-2571616 beschreibt eine Behandlung der Cellulite durch Verwendung von Östrogen und/oder Progesteron in Form einer Flüssigkeit, eines Gels oder einer Creme in homöopathischer Verdünnung.

Alle bisher verfügbaren Behandlungsmethoden der Cellulite sind jedoch nicht zufriedenstellend.

Es ist daher Aufgabe der vorliegenden Erfindung, lokal die für Cellulite typisch gestörte Struktur des subkutanen Binde-Fettgewebes zu verbessern und so die Haut wieder zu straffen und zu glätten.

Die Aufgabe wird gelöst durch ein Verfahren gemäß Anspruch 1.

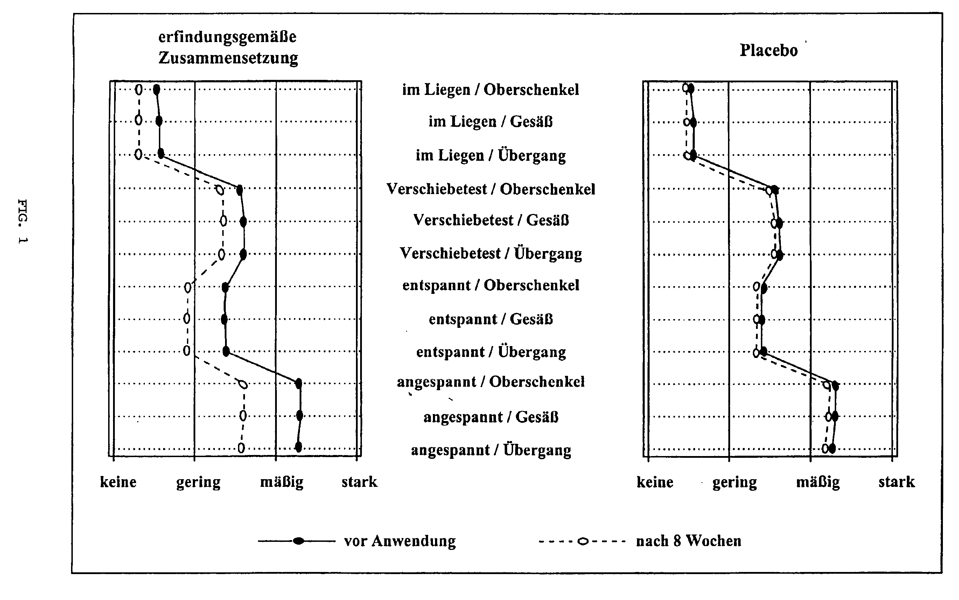

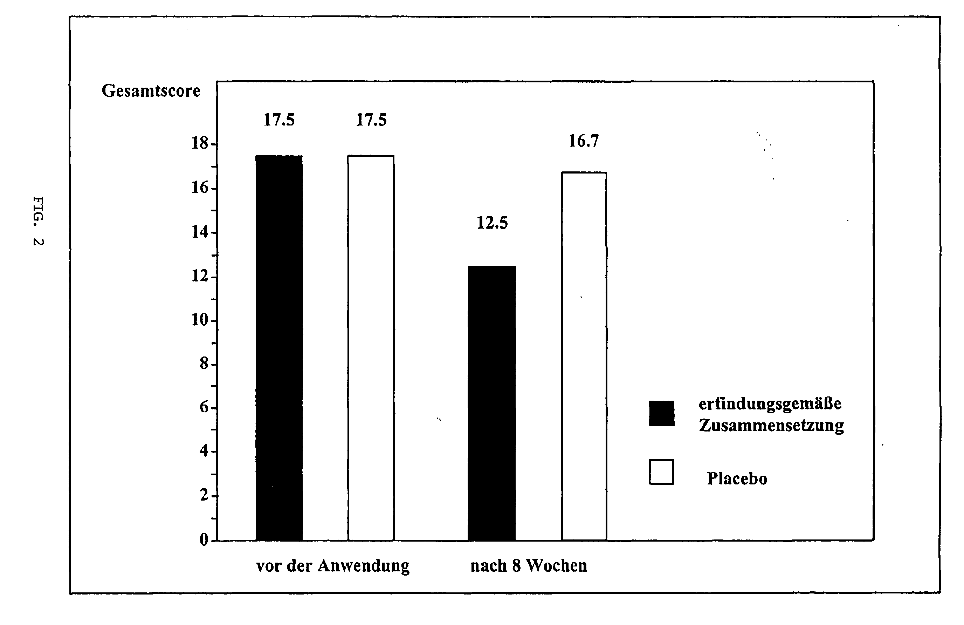

Die Figuren 1 und 2 zeigen die Wirksamkeit des erfindungsgemäßen Verahrens bei der Reduzierung der Cellulite im Vergleich zu einem Placebo, wobei Fig. 1 das Profil aller Beurteilungskriterien und Fig. 2 den Gesamtscore in den Problemzonen Oberschenkel-Gesäß zeigt.

Die Kosmetikzusammensetzung bzw. das Kosmetikum hemmt nach Applikation die Bildung und/oder die Wirkung von Östrogenen im Unterhaut-Fettgewebe (Subcutis). Bei lokaler, topischer Anwendung, d.h. Applikation der Kosmetikzusammensetzung bzw. des genannten Kosmetikums auf die Haut, in der sich das Fettgewebe befindet, wird erreicht, daß im Unterhautfettgewebe die Bildung und/oder die Wirkung der Östrogene unterdrückt wird. Dadurch kann eine günstige Umstrukturierung der Skleroproteine mit Vernetzung der Bindegewebsstränge erzielt werden. Bei der Zielgruppe der Frauen wird bei dieser Umstrukturierung bewirkt, daß bei dem "weiblichen" Typus der bindegewebigen Struktur der Unterhaut (bei der sich Bindegewebsstränge aus der Dermis steil in die Tiefe ziehen, so daß das Fettgewebe in der Subcutis in etwa in säulenförmige Kammern untergliedert ist) die Tendenz zur Bildung des "männlichen" Typus der bindegewebigen Struktur der Unterhaut entsteht. Als Folge verlaufen die Bindegewebsstraßen in der Subcutis deutlich flacher und sind stärker vernetzt als zuvor, so daß eine Vielzahl von kleineren "Fett-Kammern" entstehen. Daher verteilen sich auf die Haut einwirkende Zug- und Druckkräfte entsprechend den physikalischen Gesetzmäßigkeiten (Parallelogramm der Kräfte) auf deutlich größere Areale als vor der Applikation des genannten Stoffes. Aufgrund der Umstrukturierung treten dann Dellen oder Oberflächenunregelmäßigkeiten, wie sie durch das "Matratzenphänomen" bzw. "Orangenschalenhaut-Phänomen" umschrieben werden können, nach der Anwendung weniger oder gar nicht mehr auf. Grundlage der kosmetischen Wirkung sind die verlangsamte und verminderte Auffüllung und Neubildung von Fettzellen bei Erhaltung des physiologischen Zellumsatzes im Fettgewebe. Dies führt im Ergebnis auch zur Senkung des Füllungsgrades in den bestehenden Fettzellen des UnterhautFettgewebes. Konsekutiv führt diese "Entkrampfung" des Fettgewebes dazu, daß die gestörte lokale Durchblutung und der behinderte Transport von Lymphe und Gewebswasser (Ödeme) verbessert werden.

Der Wirkungseintritt und somit auch der Umbau der bindegewebigen Strukturen des Unterhautfettgewebes findet bei ständiger lokaler und topischer Applikation (beispielsweise 1-2mal täglich) auf die kosmetischen Problemzonen bereits innerhalb weniger Wochen statt.

Bei der Cellulite sind die Vorteile der vorliegenden Erfindung erkennbar. Die Hautoberfläche wird im Laufe der Anwendung der Kosmetikzusammensetzung bzw. des Kosmetikums, die bzw. das die Bildung und/oder die Wirkung von Östrogenen in den behandelten Fettgewebebereichen der Haut hemmt, zunehmend glatt und gestrafft, während das unerwünschte "Matratzenphänomen" stetig abnimmt. Dies zeigt sich vor allem im problematischen Hüft-, Gesäß- und Oberschenkelbereich der Frau.

Damit die beschriebene, gewünschte topische Wirkung erreicht werden kann, muß die Kosmetikzusammensetzung bzw. das Kosmetikum eine oder mehrere Substanzen umfassen, die die zugrundeliegende Hemmung der Bildung und/oder der Wirkung von Östrogenen im subkutanen Fettgewebe verursachen, wobei unter den Östrogenen alle natürliche, weibliche Sexualhormone mit östrogener Wirkung zu verstehen sind. Die im Hinblick auf Östrogene bildungs- und/oder wirkungshemmende Substanzen werden ausgewählt aus steroidalen und nicht-steroidalen Inhibitoren der Aromatase und Anti-Östrogenen, d.h. solche Substanzen, die Östrogenrezeptoren blockieren und somit als Antagonisten die Wirkung von Östrogen hemmen, und zwar Aromatase-Inhibitoren 4-Hydroxyandrost-4-en-3,17-dion, 6-Methylenandrostra-1,4-dien-3,17-dion, 10-(2-Propynyl)estr-4-en-3,17-dion und 7α-substituierte Androstendion-Derivate 6-[(4-Chlorophenyl) (1H-1,2,4-triazol-1-yl)-methyl] 1-methyl-1H-benzotriazol, 2,2'-[5-(1H-1,2,4-triazol-1-yl methyl)-1,3-phenylen]bis (2-methylproprionitril), 4-[1-(Cyanophenyl)-1-(1,2,4-triazolyl)methyl]benzonitril, (4- (5,6,7,8-Tetrahydro-imidazo-[1,5a]-pyridin-5-yl) benzonitril Monohydrochlorid und Pyridoglutethimid; und Östrogenrezeptorblocker: die Tamoxifen (Z-2-[4-(1,2-Diphenyl-1-butenyl)-phenoxy]-N,N-dimethylamin) und Aminoglutethimid (3-(4-Aminophenyl)-3-ethyl-2,6-piperidin-dion) sowie die Tamoxifen Analoga 3-Hydroxytamoxifen, 4-Hydroxytamoxifen sowie das 7 α-Alkyl-Sulfinyl-Tamoxifen-Analog.
Hinsichtlich der Bezeichnungen dieser Substanzen sowie deren Verfügbarkeit siehe beispielsweise "Rote Liste", Editio Cantor, Aulendorf (DE), (1985).

Bevorzugte Substanzen, die gemäß der Erfindung in das zu verwendende Kosmetikum hineingegeben werden, sind die der Aromatase-Inhibitoren. Es wird angenommen, daß die Aromatase-Inhibitoren über die Hemmung der lokalen Neubildung von Östrogenen in dem betreffenden Fettgewebe einen effizienten Einfluß auf den kosmetisch vorteilhaften Umbau der bindegewebigen Strukturen des Unterhautfettgewebes, wie oben beschrieben, ausübt und somit beispielsweise der Cellulite entgegenwirkt.

Solche Aromataseinhibitoren sind an sich bekannt, aber auf einem ganz anderen Gebiet, nämlich als systemisch eingesetzte Wirkstoffe zur medizinisch-therapeutischen Behandlung von Brustkrebs. In diesem Zusammenhang wird verwiesen auf die Übersichtsartikel von A.M.H. Brodi in: "J. Steorid Biochem. Molec. Biol.", Vol. 49, No. 4-6, pp. 281-287 (1994), sowie P. E. Goss und K.M.E.H. Gwyn in: "Journal of Clinical Oncology", Vol. 12, No. 11, pp. 2460-2470 (1994). Zur Bestimmung der Aromatase-Inhibition und der nachfolgenden Östrogenreduzierung wird auf die in den genannten Übersichtsartikeln angegebenen, weiteren Literaturnachweise verwiesen, s. beispielsweise A.M.H. Brodi et al. in: "J. Steroid Biochem. Molec. Biol.", Vol. 7, pp. 787-793 (1976), und D.A. Marsh et al. in: "J. Med. Chem.", Vol. 28, pp. 788-795 (1985).

Die EP-A-0 240 131 offenbart eine Tamoxifen enthaltende, pharmazeutische zusammensetzung zur topischen Anwendung gegen Psoriasis. Ansonsten sind die Anti-Östrogene bisher stets nur im Zusammenhang mit der systemisch therapeutischen Behandlung von Brustkrebs beschrieben worden.

Der Grund für die Wirkung von steroidalen und nicht-steroidalen Aromatase-Inhibitoren sowie von Östrogenrezeptorblockern zur erfindungsgemäßen Anwendung bei Cellulite wird darin vermutet, daß die lokale Inhibierung der Aromatase, also die Hemmung der Östrogenentstehung vor Ort, bzw. die Antiöstrogenwirkung zu einer dauerhaften Erniedrigung des Östrogengehalts in den subkutanen Fettzellen führt. So wird gemäß der vorliegenden Erfindung durch die lokale Anflutung der topisch eingesetzten Wirksubstanz faktisch nur die Östrogenbildung bzw. -wirkung im peripheren subkutanen Fettgewebe gehemmt. Eine androgene Wirkung ist nicht zu erwarten und konnte auch nicht nachgewiesen werden. Die erfindungsgemäß eingesetzten Substanzen wirken nur lokal, also nicht systemisch. Bei allen behandelten Frauen traten keinerlei Unverträglichkeiten auf.

Damit sich die Wirkmechanismen zur Lösung der kosmetischen Probleme gegenseitig ergänzen und günstig beeinflussen können, wird gemäß einer bevorzugten Ausführungsform ein Kosmetikum zur Applikation auf die Haut eingesetzt, welches ein oder mehrere Aromatase-Inhibitoren und ein oder mehrere Östrogenrezeptorblocker kombiniert umfaßt. Das bei der Kombination zu verwendende Mengenverhältnis ist dabei unkritisch und kann den jeweiligen Bedürfnissen angepaßt werden. So kann zum Beispiel jeweils die eine Substanzart oder die andere Substanzart überwiegen, je nachdem, welcher Wirkungsweg vorrangig angestrebt wird. Das gewichtsmäßige Mengenverhältnis von Aromatase-Inhibitor zu Östrogenrezeptorblocker liegt beispielsweise in einem Bereich von 90/10 bis 10/90, insbesondere in einem Bereich von 60/40 bis 40/60.

Die erfindungsgemäß verwendete Kosmetikzusammensetzung bzw. das erfindungsgemäß verwendete Kosmetikum mit den Östrogen-hemmenden Wirksubstanzen ist auch deshalb sehr gut für die topische Anwendung geeignet, weil die in Frage kommenden Wirksubstanzen in der Regel eine gute bis sehr gute perkutane Resorptionsfähigkeit aufweisen. Falls in einzelnen Fällen die perkutane Resorption Probleme bereitet, oder falls eine gesteigerte perkutane Resorption erreicht werden soll, können vorzugsweise zusätzlich Mittel zur Förderung der perkutanen Resorption in dem zu verwendenden Kosmetikum eingesetzt werden. Solche Mittel zur Förderung der perkutanen Resorption sind bekannt. Beispielsweise eignen sich hierfür Hyaluronidate, Dimethylsulfoxid (DMSO) und dergleichen.

Zur topischen Anwendung kann eine hierfür geeignete Formulierung des zu verwendenden Stoffs gewählt werden, z.B. eine Salbe, eine Creme, ein Gel, eine Emulsion (Lotio), ein Puder oder ein Öl etc.. Zu diesem Zweck umfaßt die Kosmetikzusammensetzung bzw. das Kosmetikum Zusatzstoffe, die für die entsprechende Formulierung als Salbe, Creme, Gel, Emulsion oder Öl üblich sind. Beschriebene sowie handelsübliche, herkömmliche Hautpflegemittel sind in den jeweiligen Formulierungen zum Einsatz in der vorliegenden Erfindung bestens geeignet. Als übliche Zusatzstoffe für solche Formulierungen dienen beispielsweise pflanzliche Öle wie Mandelöl, Olivenöl, Pfirsichkernöl, Erdnußöl, Ricinusöl u. dergl., Pflanzenextrakte, etherische Öle, Vitaminöle, Fette und fettähnliche Stoffe, Lipoide, Phosphatide, Kohlenwasserstoffe wie Paraffine, Vaseline, Lanolin, Wachse u. dergl., Detergentien, weitere Hautwirkstoffe wie Lecithin, Wollfettalkohole, Carotin u. dergl., Hautnährstoffe, Parfums, Alkohole, Glycerol, Glykole, Harnstoff, Talk, Konservierungsmittel, Sonnenschutzmittel, Farbstoffe wie Titanweiß und Zinkweiß, Antioxidantien usw.. Als Grundsubstanz dient im allgemeinen Wasser, so daß - üblicherweise unter Zusatz von Emulgatoren wie Fettalkoholsulfate, Alkaliseifen, Lecitine, Triethanolamin u. dergl. - eine O/W- oder W/O-Emulsion erhalten wird.

Die Konzentrationen der Wirksubstanz zur Hemmung der Östrogenbildung bzw. -wirkung in solchen Formulierungen sind nicht kritisch und können auf den jeweiligen Anwendungsfall angepaßt werden. Geeignet ist beispielsweise eine Wirkstoffkonzentration in der gesamten Kosmetikzusammensetzung von 0,0001 bis 10 Gewichtsprozent (Gew.%), vorzugsweise 0,001 bis 1 Gew.% und insbesondere 0,01 bis 0,5 Gew.%.

Der Gehalt des ggf. einzusetzenden Resorptionsfördermittels hängt in erster Linie von der Art des Resorptionsfördermittels ab. Die jeweils herkömmlich eingesetzten Gehaltswerte sind dabei völlig geeignet. Hyaluronidate beispielsweise können in einer Konzentration von 0,01 bis 1 Gew.%, insbesondere 0,05 bis 0,2 Gew.% verwendet werden. Für DMSO ist ein weiterer Gehaltsbereich geeignet, beispielsweise 1 bis 25 Gew.%, insbesondere 5 bis 10 Gew.%.

Die weiteren, gegebenenfalls vorhandenen Zusatzstoffe können in den für die jeweiligen Formulierungen üblichen Mengen eingesetzt werden.

Zur kosmetischen Behandlung der Cellulite-Formen, braucht das beschriebene Kosmetikum bzw. die Kosmetikzusammensetzung lediglich regelmäßig auf die zu behandelnden Hautpartien, insbesondere im Hüft-, Oberschenkel- und Gesäßbereich, aufgetragen und leicht einmassiert zu werden (beispielsweise ein- bis zweimal täglich). Durch die erfindungsgemäße Behandlungsmethode stellt sich bereits nach wenigen Wochen eine Glättung und Straffung der behandelten Hautpartien ein, ohne daß systemische Nebenwirkungen auftreten können bzw. auftreten.

Die kosmetische Behandlungsmethode gemäß der vorliegenden Erfindung gewährleistet demnach eine effektive kosmetische Wirkung, ohne daß es einer aufwendigen mechanischen Behandlung bedarf, wie es nach der eingangs beschriebenen, mechanischen Behandlungsmethoden gegen Cellulite erforderlich ist. Im Gegensatz zu diesbezüglich kritisierten, neuerdings auf dem Markt befindlichen Haut-Cellulitemitteln besitzt das erfindungsgemäße Kosmetikum eine ausgezeichnete Wirkung bei der Cellulite.

Die vorliegende Erfindung wird nachstehend anhand folgender Beispiele näher erläutert.

### Bezugsbeispiel 1

Folgende Bestandteile wurden zur Herstellung einer Creme zusammengemischt:

| | |
|---|---|
| Harnstoff | 10,0 g |
| Titanoxid | 15,0 g |
| Vaseline | 25,0 g |
| Isopropylpalmitat | 10,0 g |
| gehärtetes Erdnußöl | 10,0 g |
| Tween 80 | 5,0 g |
| Oxidierte Sojaglycine mit Aromatase-Hemmwirkung | 0,35 g |
| ger. Wasser ad. | 100,0 g |

### Klinische Überprüfung der Wirksamkeit

**Prüfdesign.** Der Einfluß der vorstehend bezeichneten Creme auf den Ausprägungsgrad des Orangenhautphänomens bei Probandinnen (24 Frauen im Alter von 20 bis 62 Jahren) mit Cellulite im Stadium II-III wurde im Rahmen einer randomisierten doppeltblinden klinischen Studie im Vergleich zu Placebo geprüft. Als Placebo diente die Cremegrundlage ohne Wirksubstanz. Die Studie wurde als halbseiten Versuch durchgeführt; d.h. jede Probandin hat eine Körperseite mit der erfindungsgemäßen Creme-Zusammensetzung behandelt und die zweite Körperseite mit Placebo. Die Zuordnung der beiden Behandlungen zur rechten oder linken Körperseite erfolgte anhand einer vor Beginn der Studie erstellten Randomisierungsliste. Beide Cremes wurden während des gesamten Studienverlaufs einmal täglich auf die betroffenen Körperregionen aufgetragen und leicht einmassiert. Über die aufzutragende Menge entschieden die Frauen nach eigenem subjektiven Empfinden. Nach der Einschlußuntersuchung erfolgten im Verlauf der Behandlung zwei Kontrolluntersuchungen im Abstand von vier Wochen. Vor Behandlungsbeginn wurde neben der Beurteilung der Cellulite eine umfassende Anamnese im Hinblick auf mögliche, die Cellulite beeinflussende Parameter erhoben.

**Beurteilungskriterien.** Zur Klassifizierung der Cellulite wurde das makroskopische Erscheinungsbild der Haut in den drei Problemzonen Oberschenkel, Gesäß und Übergangsbereich. Oberschenkel-Gesäß mittels einer vordefinierten vierstufigen Skala mit den Werten 0 = keine Orangenhaut und 1 = geringe, 2 = mäßige, 3 = starke Ausprägung des Orangenhautphänomens, beurteilt. Die Beurteilung erfolgte sowohl im Liegen als auch im Stehen bei entspannter und angespannter Muskulatur. Desweiteren wurde überprüft, ob und wie stark sich das Orangenhautphänomen durch Zusammenschieben der Haut auslösen ließ. So wurden sowohl für die mit der erfindungsgemäßen Zusammensetzung behandelte als auch für die mit dem Placebo behandelte Körperseite insgesamt 12 Beobachtungswerte dokumentiert. Für die Überprüfung der Wirksamkeit wurde ein Gesamtscore als Summe über alle 12 Beobachtungswerte gebildet.

**Ergebnisse.** Bei der Anfangsuntersuchung wurden in Bezug auf die Ausprägung der Cellulite keine Unterschiede zwischen erfindungsgemäß behandelter bzw. Placebo-behandelter Körperseite beobachtet. Die stärkste Ausprägung des Cellulite typischen Hautbildes wurde im Stehen bei angespannter Muskulatur beobachtet. In Figur 1 sind die Mittelwerte für die Beurteilung im Liegen, im Stehen bei entspannter und angespannter Muskulatur, sowie für den Verschiebetest für jede der drei Problemzonen als graphisches Profil über alle Variablen im Verlauf der Behandlung dargestellt. Die linke Grafik zeigt das Profil für die erfindungsgemäße Behandlung, die rechte Grafik das Profil für die Placebo-Behandlung mit jeweils einer Kurve für die Anfangsuntersuchung und die zweite Kontrolluntersuchung. Die Kurve für die zweite Kontrolluntersuchung der mit erfindungsgemäß behandelten Körperseite ist deutlich nach links in den unteren Wertebereich verschoben, während beide Kurven für die mit Placebo behandelte Körperseite nahezu deckungsgleich verlaufen.

**Wirksamkeitsnachweis.** Die Überprüfung der Wirksamkeit der erfindungsgemäßen Creme-Zusammensetzung basiert auf der Reduktion des Gesamtscores d.h. der Differenz des Gesamtscores bei Behandlungsbeginn minus Gesamtscore in der zweiten Kontrolluntersuchung. Die Erwartungswerte beider Behandlungsgruppen wurden varianzanalytisch nach der Methode der kleinsten Quadrate berechnet und zeigen eine deutliche (hochsignifikante p < 0.001) Überlegenheit der erfindungsgemäßen Creme (s. Figur 2).

**Verträglichkeit.** Bei keiner der Probandinnen wurde auf einer der beiden Körperseiten eine Verschlechterung des Hautbildes im Verlauf der Studie beobachtet. Klinische Auffälligkeiten, die eventuell auf eine allergische Reaktion in den behandelten Körperregionen zurückzuführen, sind, traten während der Behandlung im Rahmen der Studie nicht auf.

### Bezugsbeispiel 2

Folgende Bestandteile wurden zur Herstellung eines Gels gemischt:

| | |
|---|---|
| Ethanol 90 % | 7,0 g |
| Carbopol 934P | 0,7 g |
| Triethanolamin | 0,2 g |
| Polysorbat 80 | 5,0 g |
| Glucerol | 3,0 g |
| Oxidierte Sojaglycine mit Aromatase-Hemmwirkung | 0,35 g |
| ger. Wasser ad. | 100,0 g |

Das Gel wurde bei einer Probandin mit Cellulite, bei der sich im Stehen ein "Matratzenphänomen" zeigte, zweimal täglich (morgens und abends) auf Oberschenkel und Gesäß aufgetragen und leicht einmassiert.

Nach einer sechswöchigen Behandlungszeit erschien die Hautoberfläche an den applizierten Bereichen glatt, das "Matratzenphänomen" war beachtlich geringer ausgeprägt.

### Bezugsbeispiel 3

Folgende Bestandteile wurden zur Herstellung einer Creme zusammengemischt:

| | |
|---|---|
| Propylenglycol | 25,0 g |
| Isopropylmyristat | 6,0 g |
| Sorbitanmonostearat | 1,0 g |
| Polysorbat 80 | 2,0 g |
| Cetylstearylalkohol | 6,0 g |
| Stearylalkohol | 2,0 g |
| Glycerolmonostearat | 1,0 g |
| Hyaluronsäure | 0,1 g |
| Oxidierte Sojaglycine mit Aromatase-Hemmwirkung | 0,35 g |
| ger. Wasser ad | 100,0 g |

Die Creme wurde bei einer Probandin mit Cellulite, bei der sich im Stehen ein "Matratzenphänomen" zeigte, zweimal täglich (morgens und abends) auf Oberschenkel und Gesäß aufgetragen und leicht einmassiert.

Nach einer vierwöchigen Behandlung war die behandelte Hautoberfläche glatt und straff. Formen der Cellulite waren signifikant reduziert.

### Beispiel 4

Eine Creme wurde gemäß Bezugsbeispiel 1 hergestellt und angewandt, jedoch mit dem Unterschied, daß anstelle von 0,35 g oxidierter Sojaglycine mit Aromatase-Hemmwirkung lmg 4-Hydroxy-Tamoxifen eingesetzt wurde.

Nach der sechswöchigen Behandlung war die behandelte Oberfläche glatt und straff. Anzeichen für das "Matratzenphänomen" waren stark reduziert.

### Beispiel 5

Eine Creme wurde gemäß Bezugsbeispiel 1 hergestellt und angewandt, jedoch mit dem Unterschied, daß anstelle von 0,35 g oxidierte Sojaglycinen mit Aromatase-Hemmwirkung 0,025 g 4-Hydroxy-Tamoxifen eingesetzt wurde.

Bereits nach einer vierwöchigen Behandlung waren die Cellulite-Phänomene bei den behandelten Hautoberflächen stark reduziert.

## Patentansprüche

1. Verfahren zur kosmetischen Behandlung von Cellulite, bei dem eine Kosmetikzusammensetzung auf die zu behandelnde Haut appliziert wird, welche eine oder mehrere Substanz(en) umfaßt, die die Bildung und/oder die Wirkung von Östrogenen hemmt bzw. hemmen und die aus der nachfolgenden, aus Aromatase-Inhibitoren und Östrogenrezeptorblokkern bestehenden Gruppe ausgewählt ist bzw. sind:
Aromatase-Inhibitoren:
4-Hydroxyandrost-4-en-3,17-dion, 6-Methylenandrostra-1,4-dien-3,17-dion, 10-(2-Propynyl) estr-4-en-3,17-dion, 7α-substituierte Androstendion-Derivate; 6-[(4-Chlorophenyl) (1H-1,2,4-triazol-1-yl)-methyl]-1-methyl-1H-benzotriazol, 2,2'-[5-(1H-1,2,4-Triazol-1-yl-methyl)-1,3-phenylen]bis (2-methylproprionitril), 4-[1-(Cyanophenyl)-1-(1,2,4-triazolyl)methyl]benzonitril, 4-(5,6,7,8-Tetrahydroimidazo-[1,5a]-pyridin-5-yl)benzonitril Monohydrochlorid und Pyridoglutethimid;
Östrogenrezeptorblocker:
Aminoglutethimid (3-(4-aminophenyl)-3-ethyl-2',6-piperidine-dion), Tamoxifen und die Tamoxifen-Analoga 3-Hydroxytamoxifen, 4-Hydroxytamoxifen und das 7α-Alkylsulfinyltamoxifen-Analog.

2. Verfahren nach Anspruch 1, **dadurch gekennzeichnet, daß** die Zusammensetzung einen der genannten Aromatase-Inhibitoren umfaßt.

3. Verfahren nach Anspruch 1, **dadurch gekennzeichnet, daß** die Zusammensetzung sowohl einen der genannten Aromatase-Inhibitoren als auch einen der genannten Östrogenrezeptorblokker umfaßt.

4. Verfahren nach Anspruch 1, **dadurch gekennzeichnet, daß** die Zusammensetzung ferner/Mittel zur Förderung der perkutanen Resorption umfaßt.

5. Verfahren nach Anspruch 1, **dadurch gekennzeichnet, daß** die Zusammensetzung als salbe, Creme, Gel oder Emulsion bzw. Lotio formuliert ist.

6. Verfahren nach Anspruch 5, **dadurch gekennzeichnet, daß** die Zusammensetzung Zusatzstoffe umfaßt, die für die entsprechende Formulierung als Salbe, Creme, Gel, Emulsion bzw. Lotio üblich sind.

## Claims

1. A method for cosmetic treatment of cellulite, in the course of which a cosmetic composition is applied to the skin to be treated, which cosmetic composition comprises one or more substance(s) which inhibit(s) generation and/or effect of estrogens and which are selected from aromatase inhibitors and estrogen receptor blockers:
aromatase inhibitors:
4-hydroxyandrost-4-ene-3,17-dione, 6-methyleneandrostra-1,4-diene-3,17-dione, 10-(2-propynyl)estr-4-ene-3,17-dione, 7α-substituted androstenedione derivatives; 6-[(4-chlorophenyl)(1H-1,2,4-triazole-1-yl)-methyl]-1-methyl-1H-benzotriazole, 2,2'-[5-(1H-1,2,4-triazole-1-yl-methyl)-1,3-phenylene]bis(2-methylproprionitrile), 4-[1-(cyanophenyl)-1-(1,2,4-triazolyl)methyl]benzonitrile], (4-(5,6,7,8-tetrahydroimidazo-[1,5a]-pyridin-5-yl)benzonitrile monohydrochlorid and pyridoglutethimide;
estrogen receptor blockers:
aminoglutethimid [3-(4-aminophenyl)-3-ethyl-2,6-piperidine-dione], tamoxifen and the tamoxifen-analogues 3-hydroxytamoxifen, 4-hydroxytamoxifen und the 7α-alkylsulfinyltamoxifen; analogue.

2. The method according to Claim 1, **characterised in that** the composition comprises said aromatase inhibitor.

3. The method according to Claim 1, **characterised in that** the composition comprises both said aromatase inhibitor and said estrogen receptor blocker.

4. A method according to Claim 1, **characterised in that** the composition further comprises means for promoting percutaneous resorption.

5. A method according to Claim 1, **characterised in that** the composition is formulated as a paste, cream, gel or emulsion or lotion.

6. A method according to Claim 5, **characterised in that** the composition comprises additives which are conventionally used for respective formulation as paste, cream, gel, emulsion or lotion.

## Revendications

1. Procédé de traitement cosmétique de la cellulite, dans lequel une composition cosmétique est appliquée sur la peau à traiter, qui comprend une ou plusieurs substances qui inhibent la formation et/ou l'effet d'estrogènes et qui sont choisies dans le groupe suivant consistant en les inhibiteurs d'aromatase et les agents bloquant les récepteurs d'estrogènes :
inhibiteurs d'aromatase :
4-hydroxyandrost-4-ène-3,17-dione, 6-méthylèneandrostra-1,4-diène-3,17-dione, 10-(2-propynyl)estr-4-ène-3,17-dione, dérivés d'androstènedione 7α-substitués ; 6-[(4-chlorophényl) (1H-1,2,4-triazol-1-yl)-méthyl]-1-méthyl-1H-benzotriazole, 2,2'-[5-(1H-1,2,4-triazol-1-yl-méthyl)-1,3-phénylène]bis(2-méthylpropionitrile), 4-[1-(cyanophényl)-1-(1,2,4-triazolyl)méthyl]benzonitrile, monochlorhydrate de 4-(5,6,7,8-tétrahydroimidazo-[1,5a]-pyridin-5-yl)benzonitrile et pyridoglutéthimide ;
agents bloquant les récepteurs d'estrogènes :
aminoglutéthimide (3-(4-aminophényl)-3-éthyl-2,6-pipéridinedione), tamoxifène et les analogues du tamoxifène 3-hydroxytamoxifène, 4-hydroxytamoxifène et l'analogue 7α-alkylsulfinyltamoxifène.

2. Procédé selon la revendication 1 **caractérisé en ce que** la composition comprend l'un des inhibiteurs d'aromatase cités.

3. Procédé selon la revendication 1 **caractérisé en ce que** la composition comprend aussi bien l'un des inhibiteurs d'aromatase cités que l'un des agents bloquant les récepteurs d'estrogènes cités.

4. Procédé selon la revendication 1 **caractérisé en ce que** la composition comprend en outre des agents pour favoriser la résorption percutanée.

5. Procédé selon la revendication 1 **caractérisé en ce que** la composition est formulée sous forme de pommade, de crème, de gel ou d'émulsion ou de lotion.

6. Procédé selon la revendication 5 **caractérisé en ce que** la composition comprend des additifs qui sont courants pour la formulation correspondante sous forme de pommade, de crème, de gel ou d'émulsion ou de lotion.
